# EUROPEAN PATENT SPECIFICATION

(11) **EP 3 757 216 B1**
(45) Date of publication and mention of the grant of the patent: **07.08.2024**
(21) Application number: 19757271.2
(22) Date of filing: 21.02.2019
(51) Int. Cl.: C07K 1/22, C12N 15/70, C40B 40/08, C07K 14/735, C07K 17/00, C12N 15/10

(54) **FC-BINDING PROTEIN HAVING IMPROVED ACID STABILITY, PRODUCTION METHOD FOR SAID PROTEIN, AND ANTIBODY-ADSORBING AGENT USING SAID PROTEIN**
FC-BINDENDES PROTEIN MIT VERBESSERTER SÄURESTABILITÄT, VERFAHREN ZUR HERSTELLUNG DIESES PROTEINS UND ANTIKÖRPER-ADSORBIERENDES MITTEL, DAS DIESES PROTEIN VERWENDET
PROTÉINE DE LIAISON À FC AYANT UNE STABILITÉ AMÉLIORÉE VIS-À-VIS DES ACIDES, PROCÉDÉ DE PRODUCTION DE LADITE PROTÉINE ET AGENT D'ADSORPTION D'ANTICORPS UTILISANT LADITE PROTÉINE

(30) Priority: 22.02.2018 JP 2018029994; 20.08.2018 JP 2018154007; 11.01.2019 JP 2019003038
(43) Date of publication of application: 30.12.2020
(73) Proprietor: Tosoh Corporation, Yamaguchi 746-8501 (JP)
(72) Inventor: TAKAYAMA, Masumi, Ayase-shi, Kanagawa 252-1123 (JP); TERAO, Yosuke, Ayase-shi, Kanagawa 252-1123 (JP)
(74) Representative: Vigand, Philippe
(86) International application number: PCT/JP2019/006640
(87) International publication number: WO 2019/163919

(56) References cited:
- EP-A1- 2 546 341
- WO-A1-2011/111393
- WO-A1-2015/041303
- WO-A1-2015/041303
- WO-A1-2015/199154
- WO-A1-2018/056374
- WO-A2-2018/151817
- JP-A- 2017 178 908
- DATABASE Geneseq [online] 16 November 2017 (2017-11-16), "Human modified recombinant Fc gamma RIIb protein mutant, SEQ ID 4.", XP002804717, retrieved from EBI accession no. GSP:BEJ16147 Database accession no. BEJ16147
- MENENDEZ-ARIAS, LUIS ET AL.: "Engineering protein thermal stability: Sequence statistics point to residue substitutions in alpha-helices", J. MOL. BIOL., vol. 206, 1989, pages 397 - 406, XP000611200, doi:10.1016/0022-2836(89)90488-9

## Description

### FIELD

The present invention relates to an Fc-binding protein having binding affinity to immunoglobulin G (IgG). More specifically, the present invention relates to an Fc-binding protein with improved acid stability compared to wild-type Fcy receptor-IIa, obtained by substituting an amino acid residue with another amino acid residue, a method for producing the protein, and an antibody adsorbent obtained by immobilizing the protein on an insoluble carrier.

### BACKGROUND

Fc receptors are receptor proteins that bind to the Fc region of immunoglobulin molecules and perform intracellular signaling on binding to an antigen-immunoglobulin immune complex (NPL 1). The particularly important Fcy receptor (FcyR), which binds to the immunoglobulin G (IgG), can be subdivided into the following subgroups: FcyRI (CD64), FcyRIIa (CD32a), FcyRIIb (CD32b), FcyRIIc (CD32c), FcyRIIIa (CD16a) and FcyRIIIb (CD16b) (NPL 1 and 2). Binding of the IgG immune complex to FcyR occurs due to binding of the Fc region of IgG to the FcyR. Each type of FcyR molecule has an IgG Fc region recognition domain which recognizes one type of IgG or IgGs belonging to the same subclass. This determines which accessory cells are recruited for individual immune responses (NPL 1 and 2). Document JP2017 178908 A describes a substitution Leu190Ser in a FcyRIIb. Document WO2015041303 describes FcyRIIIa variants having higher stability against heat and acid than wild type protein. Document EP2546341 discloses FcyRI variants, having higher stability against heat and acid than wild type protein.

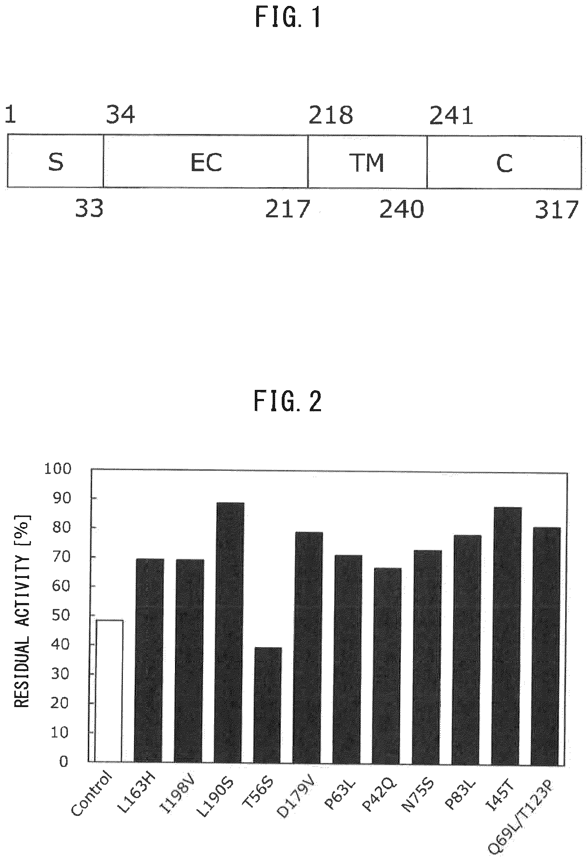

Among the FcyRs, FcyRIIa is present on macrophages, neutrophils, etc., and is involved in ADCP (antibody-dependent cellular phagocytosis) action which is an important human immune mechanism (NPL 3). The amino acid sequence of human FcyRIIa (SEQ ID NO: 1) is registered in public databases such as UniProt (Accession Number: P12318). Furthermore, the functional domain, the signal peptide sequence for traversing the cell membrane, and the position of the transmembrane domain of the human FcyRIIa structure are similarly publicly available. FIG. 1 shows a schematic structure of FcyRIIa. Note that the amino acid numbers in FIG. 1 correspond to the amino acid numbers set forth in SEQ ID NO: 1. That is, in SEQ ID NO: 1, the methionine at position 1 to the serine at position 33 constitute a signal sequence (S), the glutamine at position 34 to the glycine at position 217 constitute an extracellular domain (EC), the isoleucine at position 218 to the tyrosine at position 240 constitute a transmembrane domain (TM), and the cysteine at position 241 to the asparagine at position 317 constitute an intracellular domain (C).

In order to make the FcyRIIa industrially applicable, it is preferable for the acid stability thereof to be high in view of use, storage, and the like. However, there has heretofore been no attempt to improve acid stability of human FcyRIIa.

### [CITATION LIST]

### [PATENT LITERATURE]

[NPL 1] Takai T., Jpn. J. Clin. Immunol., 28, 318-326, 2005
[NPL 2] J. Galon et al., Eur. J. Immunol., 27, 1928-1932, 1997
[NPL 3] J. O. Richards et al., Mol. Cancer Ther., 7, 2517-2527, 2008

### SUMMARY

### [TECHNICAL PROBLEM]

The object of the present invention is to provide an Fc-binding protein with improved acid stability compared to wild type human FcyRIIa, a method for producing the protein, and an antibody adsorbent using the protein.

### [SOLUTION TO PROBLEM]

As a result of extensive research carried out by the present inventors to achieve the above object, the amino acid residues involved in improving acid stability in human FcyRIIa were identified and variants in which an amino acid residue was substituted with another amino acid were found to have excellent acid stability. Thereby the present invention was achieved.

The present invention is defined by the claims.

Specifically, the present application encompasses the following embodiments [1] to [9].
[1] An FcyRIIa protein comprising at least amino acid residues from glutamine at position 29 to glutamine at position 201 of the amino acid sequence set forth in SEQ ID NO: 2, provided the FcyRIIa protein has
   a substitution of leucine to serine at position 190 of SEQ ID NO: 2, and
   amino acid substitutions at the 6 positions indicated below:
   a substitution of isoleucine to valine at position 68 of SEQ ID NO: 2,
   a substitution of histidine to glutamine at position 80 of SEQ ID NO: 2,
   a substitution of serine to threonine at position 84 of SEQ ID NO: 2,
   a substitution of asparagine to threonine at position 90 of SEQ ID NO: 2,
   a substitution of asparagine to serine at position 91 of SEQ ID NO: 2,
   a substitution of histidine to arginine at position 125 of SEQ ID NO: 2.
[2] The Fc-binding protein according to [1] selected from (iv), (vii) or (x) below:
   (iv) an FcyRIIa protein comprising at least amino acid residues from glutamine at position 29 to glutamine at position 201 of an amino acid sequence set forth in any of SEQ ID NOs: 5, 13, 17, 19, 23, and 25;
   (vii) an FcyRIIa protein comprising at least amino acid residues from glutamine at position 29 to glutamine at position 200 of the amino acid sequence set forth in SEQ ID NO: 35;
   (x) an FcyRIIa protein comprising at least amino acid residues from glutamine at position 29 to glutamine at position 196 of the amino acid sequence set forth in SEQ ID NO: 43.
[3] A polynucleotide encoding the FcyRIIa protein according to any one of [1] or [2].
[4] An expression vector comprising the polynucleotide according to [3].
[5] A transformant obtainable by transforming a host with the recombinant vector according to [4] and capable of producing an FcyRIIa protein.
[6] The transformant according to [5], wherein the host is *Escherichia coli.*
[7] A method for producing an FcyRIIa protein, comprising the steps of: producing an FcyRIIa protein by culturing the transformant according to [5] or [6]; and harvesting the FcyRIIa protein from the obtained culture product.
[8] An antibody adsorbent obtainable by immobilizing the FcyRIIa protein according to any one of [1] or [2] on an insoluble carrier.
[9] An antibody separation method comprising the steps of: adding a solution containing an antibody to a column filled with the adsorbent according to [8] and adsorbing the antibody to the adsorbent; and eluting the antibody adsorbed to the adsorbent using an eluent.

The present invention will be described in detail below.

The FcyRIIa protein of the present invention is a protein having antibody Fc regionbinding properties and comprising at least the amino acid residues from the glutamine at position 29 to the glutamine at position 201 of a polypeptide comprising a wild type human FcyRIIa extracellular domain consisting of the amino acid sequence set forth in SEQ ID NO: 2, provided the protein has the amino acid substitutions at specific positions in the amino acid residues from position 29 to position 201 as mentioned above. Note that in SEQ ID NO: 2, position 1 to position 26 constitute a MalE signal peptide (oligopeptide consisting of the amino acid residues from position 1 to position 26 of UniProt No. P0AEX9), the methionine at position 27 and the glycine at position 28 constitute a linker sequence, position 29 to position 201 constitute the FcyRIIa extracellular domain (the region from position 34 to position 206 in SEQ ID NO: 1), position 202 and position 203 constitute a glycine linker, and position 204 to position 209 constitute a histidine tag. Thus, the Fc-binding protein of the present invention may comprise the entirety or a part of the extracellular domain N-terminal side signal peptide domain or may comprise the entirety or a part of the extracellular domain C-terminal side transmembrane domain and the extracellular domain.

The Fc-binding protein of the present invention is an FcyRIIa protein comprising at least a Leu190Ser amino acid substitution since the Leu190Ser amino acid substitution particularly improves acid stability.

It is satisfactory for the FcyRIIa protein of the present invention to have amino acid substitutions at the above specific position, and disclosed but not part of the invention, provided antibody binding activity is exhibited, may further include at least one of a substitution, deletion, insertion, or addition of an amino acid residue, in addition to the above amino acid substitutions at the specified positions. In cases where an amino acid residue is deleted, a deletion that removes a loop region in the protein secondary structure improves heat resistance and is thus preferable (Manandez-Alias and P. Argos, J. Mol. Biol., 206, 1989).

The amino acid substitutions, Ile68Val, His80Gln, Ser84Thr, Asn90Thr, Asn91Ser, and His125Arg, are amino acid substitutions for improving heat stability and productivity in recombinant transformants (WO2018/056374). Thus, by further including amino acid substitutions (Ile68Val, His80Gln, Ser84Thr, Asn90Thr, Asn91Ser, and His125Arg) at the six positions in the FcyRIIa protein of the present invention having the at least one acid substitution in the specific position, an FcyRIIa protein with improved acid stability, heat stability, and productivity in recombinant transformants compared to wild type FcyRIIa can be obtained.

A FcyRIIa protein disclosed, but not part of the invention may also have conservative amino acid substitutions wherein either or both of the physical and chemical properties of both amino acids are similar. It is known by a person skilled in the art that, generally, without being limited to Fc-binding proteins, proteins which have undergone a conservative substitution retain the function of the proteins which have not undergone the conservative substitution. Examples of conservative substitutions include substitutions between glycine and alanine, aspartic acid and glutamic acid, serine and proline, or glutamic acid and alanine (Protein Structure and Function, Medical Science International, 9, 2005).

Further, the FcyRIIa protein of the present invention may further have an oligopeptide added to the N-terminal side or C-terminal side thereof which is useful for separating the protein from a solution containing contaminants. The oligopeptide may be polyhistidine, polylysine, polyarginine, polyglutamic acid, or polyaspartic acid. Further, the FcyRIIa protein of the present invention may further have, added thereto on the N-terminal side or C-terminal side thereof, an oligopeptide containing cysteine which is useful for immobilization of the FcyRIIa protein of the present invention to a solid phase such as a support for chromatography. The length of oligopeptides added to the N-terminal or C-terminal side of the FcyRIIa protein is not particularly limited provided the stability of the FcyRIIa protein and the ability thereof to bind IgG are not impaired. When adding an oligopeptide to the FcyRIIa protein of the present invention, a method well known to a person skilled in the art may be used to add the oligopeptide by means of genetic engineering using a previously created polynucleotide encoding the oligopeptide to the N-terminal or C-terminal side of the FcyRIIa protein. Alternatively, a chemically synthesized oligopeptide may be chemically bonded to the N-terminal or C-terminal side of the FcyRIIa protein of the present invention. Further, a signal peptide for effectively promoting expression in a host cell can be added to the N-terminal side of the FcyRIIa protein of the present invention. Examples of the signal peptide when *E. coli* is the host include PelB, DsbA, MalE (the region from position 1 to position 26 of the amino acid sequence set forth in UniProt No. P0AEX9), and TorT which are signal peptides that secrete proteins in the periplasm (Japanese Unexamined Patent Publication (Kokai) No. 2011-097898).

Preferred embodiments of the FcyRIIa protein of the present invention include Fc-binding proteins containing at least a polypeptide consisting of any of the amino acid sequences indicated in (a), (c) to (i) below. These FcyRIIa proteins are preferable in terms of improved acid stability (acid resistance).

### (a) FcyRIIa-m7 (the amino acid residues from position 29 to position 201 of the amino acid sequence set forth in SEQ ID NO: 5)

A polypeptide comprising the amino acid residues from the glutamine at position 29 to the glutamine at position 201 of the amino acid sequence set forth in SEQ ID NO: 2, provided the polypeptide has the following amino acid substitutions in the amino acid residues from position 29 to position 201: Ile68Val, His80Gln, Ser84Thr, Asn90Thr, Asn91Ser, His125Arg, and Leu190Ser.

### (c) FcyRIIa-m8B (the amino acid residues from position 29 to position 201 of the amino acid sequence set forth in SEQ ID NO: 13)

A polypeptide comprising the amino acid residues from the glutamine at position 29 to the glutamine at position 201 of the amino acid sequence set forth in SEQ ID NO: 2, provided the polypeptide has the following amino acid substitutions in the amino acid residues from position 29 to position 201: Ile68Val, Gln69Leu, His80Gln, Ser84Thr, Asn90Thr, Asn91Ser, His125Arg, and Leu190Ser.

### (d) FcyRIIa-m8D (the amino acid residues from position 29 to position 201 of the amino acid sequence set forth in SEQ ID NO: 17)

A polypeptide comprising the amino acid residues from the glutamine at position 29 to the glutamine at position 201 of the amino acid sequence set forth in SEQ ID NO: 2, provided the polypeptide has the following amino acid substitutions in the amino acid residues from position 29 to position 201: Ile68Val, His80Gln, Ser84Thr, Asn90Thr, Asn91Ser, Thr123Pro, His125Arg, and Leu190Ser.

### (e) FcγRIIa-m10A (the amino acid residues from position 29 to position 201 of the amino acid sequence set forth in SEQ ID NO: 19)

A polypeptide comprising the amino acid residues from the glutamine at position 29 to the glutamine at position 201 of the amino acid sequence set forth in SEQ ID NO: 2, provided the polypeptide has the following amino acid substitutions in the amino acid residues from position 29 to position 201: Ile45Thr, Ile68Val, Gln69Leu, His80Gln, Ser84Thr, Asn90Thr, Asn91Ser, Thr123Pro, His125Arg, and Leu190Ser.

### (f) FcγRIIa-m10C (the amino acid residues from position 29 to position 201 of the amino acid sequence set forth in SEQ ID NO: 23)

A polypeptide comprising the amino acid residues from the glutamine at position 29 to the glutamine at position 201 of the amino acid sequence set forth in SEQ ID NO: 2, provided the polypeptide has the following amino acid substitutions in the amino acid residues from position 29 to position 201: Ile68Val, Gln69Leu, His80Gln, Pro83Leu, Ser84Thr, Asn90Thr, Asn91Ser, Thr123Pro, His125Arg, and Leu190Ser.

### (g) FcγRIIa-m11 (the amino acid residues from position 29 to position 201 of the amino acid sequence set forth in SEQ ID NO: 25)

A polypeptide comprising the amino acid residues from the glutamine at position 29 to the glutamine at position 201 of the amino acid sequence set forth in SEQ ID NO: 2, provided the polypeptide has the following amino acid substitutions in the amino acid residues from position 29 to position 201: Ile45Thr, Ile68Val, Gln69Leu, His80Gln, Pro83Leu, Ser84Thr, Asn90Thr, Asn91Ser, Thr123Pro, His125Arg, and Leu190Ser.

### (h) FcγRIIa-m11-Del1 (the amino acid residues from position 29 to position 200 of the amino acid sequence set forth in SEQ ID NO: 35)

A polypeptide comprising the amino acid residues from the glutamine at position 29 to the glutamine at position 200 of the amino acid sequence set forth in SEQ ID NO: 2, provided the polypeptide has, in the amino acid residues from position 29 to position 200, a deletion Ser62 and the following amino acid substitutions: Ile45Thr, Ile68Val, Gln69Leu, His80Gln, Pro83Leu, Ser84Thr, Asn90Thr, Asn91Ser, Thr123Pro, His125Arg, and Leu190Ser.

### (i) FcγRIIa-m11-Del5 (the amino acid residues from position 29 to position 196 of the amino acid sequence set forth in SEQ ID NO: 43)

A polypeptide comprising the amino acid residues from glutamine at position 29 to glutamine at position 196 of the amino acid sequence set forth in SEQ ID NO: 2, provided the polypeptide has, in the amino acid residues from position 29 to position 196, deletions Arg61 to Ser65 and the following amino acid substitutions: Ile45Thr, Ile68Val, Gln69Leu, His80Gln, Pro83Leu, Ser84Thr, Asn90Thr, Asn91Ser, Thr123Pro, His125Arg, and Leu190Ser.

Note that in the FcyRIIa proteins set forth in SEQ ID NOs: 5, 13, 17, 19, 23, and 25, the methionine at position 1 to the alanine at position 26 constitute a MalE signal peptide, the methionine at position 27 and the glycine at position 28 constitute a linker sequence, the glutamine at position 29 to the glutamine at position 201 constitute an amino acid sequence of a polypeptide (FcyRIIa-m7 (SEQ ID NO: 5), FcyRIIa-m8b (SEQ ID NO: 13), FcyRIIa-m8d (SEQ ID NO: 17), FcγRIIa-m10a (SEQ ID NO: 19), FcγRIIa-m10c (SEQ ID NO: 23) or FcγRIIa-m11 (SEQ ID NO: 25)) capable of binding to the antibody Fc region, the glycines at position 202 and position 203 constitute a linker sequence, and the histidines from position 204 to position 209 constitute a tag sequence.

Note that in the FcyRIIa protein set forth in SEQ ID NO: 35, the methionine at position 1 to the alanine at position 26 constitute a MalE signal peptide, the methionine at position 27 and the glycine at position 28 constitute a linker sequence, the glutamine at position 29 to the glutamine at position 200 constitute an amino acid sequence of a polypeptide (FcγRIIa-m11-Del1) capable of binding to the antibody Fc region, the glycines at position 201 and position 202 constitute a linker sequence, and the histidines from position 203 to position 208 constitute a tag sequence.

Note that in the FcyRIIa protein set forth in SEQ ID NO: 43, the methionine at position 1 to the alanine at position 26 constitute a MalE signal peptide, the methionine at position 27 and the glycine at position 28 constitute a linker sequence, the glutamine at position 29 to the glutamine at position 196 constitute an amino acid sequence of polypeptide (FcγRIIa-m11-Del5) capable of binding to the antibody Fc region, the glycines at position 197 and position 198 constitute a linker sequence, and the histidines from position 199 to position 204 constitute a tag sequence.

Examples of methods for producing a polynucleotide encoding the FcyRIIa protein of the present invention (hereinafter referred to simply as the polynucleotide of the present invention) include:
(1) a method of converting the amino acid sequence of the FcyRIIa protein of the present invention to a nucleotide sequence and artificially synthesizing a polynucleotide comprising the nucleotide sequence; or
(2) a method of directly preparing a polynucleotide containing the whole or partial sequence of the FcyRIIa protein artificially or preparing the polynucleotide from cDNA of the FcyRIIa protein using a DNA amplification method such as PCR, followed by linkage of the prepared polynucleotide using a suitable method.

In the method (1) above, when converting the amino acid sequence to a nucleotide sequence, it is preferable that the conversion is performed in view of codon usage frequency of the transformed host. For example, if the host is *Escherichia coli,* the usage frequency of each of AGA/ AGG/ CGG/ CGA for arginine (Arg), ATA for isoleucine (Ile), CTA for leucine (Leu), GGA for glycine (Gly), CCC for proline (Pro) is low (so called rare codons) and so these codons should be avoided when performing the conversion. Public databases (for example, the Codon Usage Database on the Kazusa DNA Research Institute website) can be used to perform codon usage frequency analysis.

When introducing mutations into the polynucleotide of the present invention, error prone PCR may be used. The reaction conditions for the error prone PCR are not particularly limited, provided desired mutations can be introduced into the polynucleotide encoding the human FcyRIIa. For example, mutations can be introduced into the polynucleotide by adding the four basic types of deoxynucleotide (dATP/ dTTP/ dCTP/ dGTP) in uneven concentrations and MnCl₂ at a concentration of 0.01 to 10 mM (preferably 0.1 to 1 mM) to a PCR reaction liquid and performing PCR. Methods of introducing mutations other than error prone PCR include a method of subjecting the polynucleotide containing the whole or partial sequence of human FcyRIIa to contact with or action of an agent serving as a mutagen or ultraviolet radiation to introduce and prepare mutations. The agent used as a mutagen in the above method may be any of mutagenic agents that are usually used by a person skilled in the art such as hydroxylamine, N-methyl-N'-nitro-N-nitrosoguanidine, nitrous acid, sulfurous acid, and hydrazine.

When transforming a host using the polynucleotide of the present invention, the polynucleotide of the present invention itself may be used, but it is more preferable to use an expression vector (e.g., bacteriophages, cosmids, or plasmids commonly used for transforming prokaryotic or eukaryotic cells) into which the polynucleotide of the present invention has been introduced at a suitable site. Note that there are no limitations on the expression vector, provided the expression vector is stable and can replicate within the host to be transformed. When *E*. *coli* is used as the host, examples of the vector include a pET plasmid vector, pUC plasmid vector, pTrc plasmid vector, pCDF plasmid vector, and pBBR plasmid vector.

Further, the "suitable site" refers to a site in the expression vector that does not destroy regions related to a replication function, preferred antibiotic marker, or transmissibility. When introducing the polynucleotide of the present invention into the expression vector, it is preferable that the polynucleotide is introduced so as to be linked to a functional polynucleotide such as a promoter necessary for expression. Examples of the promoter when the host is *E. coli* include the trp promoter, tac promoter, trc promoter, lac promoter, T7 promoter, recA promoter, Ipp promoter, in addition to λ PL promoter and λ PR promoter of λ phage.

Transformation of a host using an expression vector (hereinafter referred to as expression vector of the present invention) into which the polynucleotide of the present invention has been introduced prepared by the above method, may be performed by any method commonly used by a person skilled in the art. For example, when microorganisms belonging to the *Escherichia* genus (e.g., *E. coli* JM109, *E. coli* BL21 (DE3), and *E*. *coli* W3110) are selected, transformation may be carried out by any of the methods set forth in publicly available documents (e.g., Molecular Cloning, Cold Spring Harbor Laboratory, 256, 1992). Transformants obtained by the above method are screened by a suitable method, whereby transformants (hereinafter referred to as transformants of the present invention) that can express the Fc-binding protein of the present invention can be obtained. Note that there are no particular limitations on the host for expressing the Fc-binding protein of the present invention. Examples include animal cells (CHO (Chinese Hamster Ovary) cells, HEK cells, Hela cells, COS cells, etc.), yeast (*Saccharomyces cerevisiae, Pichia pastoris, Hansenula polymorpha, Schizosaccharomyces japonicus, Schizosaccharomyces octosporus, Schizosaccharomyces pombe,* etc.), insect cells (Sf9, Sf21, etc.), *Escherichia coli* (JM109, BL21(DE3), W3110, etc.) and *Bacillus subtilis.* Note that using animal cells or *E. coli* as the host is preferable in terms of productivity and using *E. coli* as the host is more preferable.

For the preparation of the expression vector of the present invention from the transformant of the present invention, the expression vector may be prepared using the alkali extraction method or a commercially available extraction kit such as QIAprep Spin Miniprep kit (QIAGEN N.V.) from a culture product obtained by culturing the transformant of the present invention. The FcyRIIa protein of the present invention can be prepared by culturing the transformant of the present invention and harvesting the FcyRIIa protein of the present invention from the obtained culture product. Note that herein, the culture product also includes the culture medium used for culturing in addition to the cultured transformed cells of the present invention themselves. The transformant used in the protein production method of the present invention may be cultured in a culture medium appropriate for the designated host, and when the host is *E. coli,* an example of a preferred culture medium is LB (Luria-Bertani) culture medium supplemented with a necessary nutritional source. Note that in order to selectively grow the transformant of the present invention due to the presence or absence of introduction of the vector of the present invention, it is preferable to perform culturing in a culture medium having added thereto an antibiotic which corresponds to an antibiotic resistance gene contained in the vector. For example, if the vector contains a kanamycin resistance gene, kanamycin may be added to the culture medium. Further, in addition to sources of carbon, nitrogen, and inorganic salts, suitable nutritional sources may be added including one or more reducing agents selected from the group consisting of glutathione, cysteine, cystamine, thioglycolate and dithiothreitol as desired. Furthermore, a reagent such as glycine that promotes the secretion of proteins from the transformant into the culture medium may be added, specifically, if the host is *E*. *coli,* it is preferable for not more than 2% (w/v) of glycine to be added to the culture medium. If the host is *E. coli,* the culture temperature is generally 10 °C to 40 °C, preferably 20 °C to 37 °C, more preferably about 25 °C but can be selected according to the characteristics of the expressed protein. If the host is *E*. *coli,* the pH of the culture medium is set to pH 6.8 to pH 7.4 and preferably around pH 7.0. Further, if the vector of the present invention contains an inducible promoter, it is preferable that the promoter is induced under conditions which favor the expression of the FcyRIIa protein of the present invention. An example of an inducing agent is IPTG (isopropyl-β-D-thiogalactopyranoside). If the host is *E. coli,* expression of the Fc-binding protein can be induced by measuring the turbidity of the culture solution (absorbance at 600 nm), adding a suitable amount of IPTG when the turbidity is around 0.5 to 1.0, and then continuing culturing. The concentration of the IPTG added may be appropriately selected from a range of 0.005 to 1.0 mM but is preferably in a range of 0.01 to 0.5 mM. Various conditions related to IPTG induction may be selected from well-known conditions in this technical field.

In order to harvest the FcyRIIa protein of the present invention from a culture product obtained by culturing the transformant of the present invention, a method suited to the expression form of the FcyRIIa protein of the present invention in the transformant of the present invention may be used to separate/ purify the FcyRIIa protein of the present invention from the culture product to harvest the Fc-binding protein. For example, if the FcyRIIa protein is expressed in the supernatant of the culture medium, centrifugation may be performed to separate the protein from the bacterial cells and the FcyRIIa protein of the present invention may be purified from the obtained culture supernatant. Further, if the FcyRIIa protein is expressed intracellularly (including the periplasm), the bacterial cells can be collected by centrifugation, the cells can be disrupted by the addition of an enzyme treatment agent or a surfactant and the FcyRIIa protein of the present invention can be extracted and then purified. In order to purify the FcyRIIa protein of the present invention, publicly known methods in the technical field, for example, separation/ purification using liquid chromatography may be used. Liquid chromatography includes ion exchange chromatography, hydrophobic interaction chromatography, gel filtration chromatography, and affinity chromatography, and by combining such chromatographic methods when carrying out purification, the FcyRIIa protein of the present invention can be prepared to a high degree of purity. Methods for measuring the binding activity of the obtained Fc-binding protein of the present invention to IgG include, for example, measurements of the binding activity to IgG using an enzyme-linked immunosorbent assay (hereinafter referred to as ELISA) or surface plasmon resonance. For the IgG used for measuring binding activity, human IgG is preferable, and any of human IgG1, human IgG2, human IgG3, or human IgG4 may be used.

The adsorbent of the present invention can be prepared by binding the FcyRIIa protein of the present invention to an insoluble carrier. There are no particular limitations on the insoluble carrier which may include, for example: carriers made from polysaccharides, such as agarose, alginate (alginic acid salt), carrageenan, chitin, cellulose, dextrin, dextran, and starch; carriers made from synthetic polymers, such as polyvinyl alcohol, polymethacrylate, poly(2-hydroxyethylmethacrylate), and polyurethane; and carriers made from ceramics such as silica. Thereamong, carriers made from polysaccharides or synthetic polymers are preferable as the insoluble carrier. Examples of the preferable carrier include hydroxylated polymethacrylate gels such as Toyopearl (manufactured by Tosoh Corporation), agarose gels such as Sepharose (manufactured by GE Healthcare), and cellulose gels such as Cellufine (manufactured by JNC). There are no limitations to the form of the insoluble carrier which may be granular, non-granular, porous, or non-porous.

In order to immobilize the FcyRIIa protein onto the insoluble carrier, an activating group such as an N-hydroxysuccinimide (NHS) activated ester group, an epoxy group, a carboxyl group, a maleimide group, a haloacetyl group, a tresyl group, a formyl group, or a haloacetamide is provided on the insoluble carrier and immobilization may be achieved by covalent bonding between the FcyRIIa protein and the insoluble carrier via the activating group. Commercially available carriers provided with an activating group may be used as they are or may be prepared by introducing an activating group to the surface of a carrier under appropriate reaction conditions. Examples of commercially available carriers provided with an activating group include TOYOPEARL AF-Epoxy-650M, TOYOPEARL AF-Tresyl-650M (both manufactured by Tosoh Corp.), HiTrap NHS-activated HP Columns, NHS-activated Sepharose 4Fast, Flow, Epoxy-activated Sepharose 6B (both manufactured by GE Healthcare), SulfoLink Coupling Resin (manufactured by Thermo Scientific).

Meanwhile, examples of a method for introducing an activating group onto the carrier surface include a method of reacting a hydroxy group, epoxy group, carboxyl group, or amino group on the surface of the carrier with one of at least two active sites of a compound. Examples thereof include compounds that introduce an epoxy group to a hydroxy group or amino group of the carrier surface, and examples of the compounds include epichlorohydrin, ethanediol diglycidyl ether, butanediol diglycidyl ether, and hexanediol diglycidyl ether. Examples of compounds that introduce carboxyl groups to the carrier surface after the above compound has introduced an epoxy group to the carrier surface include 2-mercaptoacetic acid, 3-mercaptopropionic acid, 4-mercaptobutyric acid, 6-mercaptobutyric acid, glycine, 3-aminopropionic acid, 4-aminobutyric acid, and 6-aminohexanoic acid.

Examples of a compound that introduces a maleimide group onto a hydroxyl group, an epoxy group, a carboxyl group, or an amino group existing on the surface of a carrier include N-(ε-maleimidocaproic acid) hydrazide, N-(ε-maleimidopropionic acid) hydrazide, 4-(4-N-maleimidophenyl)acetic acid hydrazide, 2-aminomaleimide, 3-aminomaleimide, 4-aminomaleimide, 6-aminomaleimide, 1-(4-aminophenyl)maleimide, 1-(3-aminophenyl)maleimide, 4-(maleimido)phenylisocyanate, 2-maleimidoacetic acid, 3-maleimidopropionic acid, 4-maleimidobutyric acid, 6-maleimidohexanoic acid, N-(α-maleimidoacetoxy)succinimide ester, (m-maleimidobenzoyl) N-hydroxysuccinimide ester, succinimidyl-4-(maleimidomethyl)cyclohexane-1-carbonyl-6-aminohexanoic acid, succinimidyl-4-(maleimidomethyl)cyclohexane-1-carboxylic acid, (p-maleimidobenzoyl) N-hydroxysuccinimide ester, (m-maleimidobenzoyl) N-hydroxysuccinimide ester.

Examples of a compound that introduces a haloacetyl group onto a hydroxyl group or an amino group existing on the surface of the carrier include chloroacetic acid, bromoacetic acid, iodoacetic acid, chloroacetic acid chloride, bromoacetic acid chloride, bromoacetic acid bromide, chloroacetic acid anhydride, bromoacetic acid anhydride, iodoacetic anhydride, 2-(iodoacetamido)acetic acid-N-hydroxysuccinimide ester, 3-(bromoacetamido)propionic acid-N-hydroxysuccinimide ester, 4-(iodoacetyl)aminobenzoic acid-N-hydroxysuccinimide ester. Note that the examples further include a method of reacting hydroxyl groups and amino groups existing on the surface of the carrier with ω-alkenyl alkane glycidyl ether then activating the ω-alkenyl moiety by halogenation with a halogenating agent. Examples of the ω-alkenyl alkane glycidyl ether include allyl glycidyl ether, 3-butenyl glycidyl ether and 4-pentenyl glycidyl ether, and examples of halogenating agents include N-chlorosuccinimide, N-bromosuccinimide and N-iodosuccinimide.

Another example of a method for introducing an activating group onto the carrier surface includes a method of introducing an activating group to a carboxyl group existing on the surface of the carrier by using a condensing agent and an additive. Examples of the condensing agent include 1-ethyl-3-(3-dimethylaminopropyl) carbodiimide (EDC), dicyclohexyl carbodiamide and carbonyldiimidazole. Further, examples of additives include N-hydroxysuccinimide (NHS), 4-nitrophenol and 1-hydroxybenzotriazole.

Examples of a buffer solution used when immobilizing the FcyRIIa protein of the present invention on an insoluble carrier include acetate buffer, phosphate buffer, MES (2-morpholinoethane sulfonic acid) buffer, HEPES (2-[4-(2-Hydroxyethyl)-1-piperazinyl]ethansulfonic acid buffer, Tris buffer, and borate buffer. The reaction temperature during immobilization is suitably set within the temperature range of 5 °C to 50 °C in view of the reactivity of the activating group and stability of the Fc-binding protein of the present invention, and is preferably within the range of 10 °C to 35 °C.

In order to purify an antibody using the adsorbent of the present invention obtained by immobilizing the FcyRIIa protein of the present invention on the insoluble carrier, for example, a buffer solution containing the antibody is added to a column packed with the adsorbent of the present invention using a pump or other means to transfer liquid, whereby the antibody specifically binds to the adsorbent of the present invention, and thereafter the antibody can be eluted from the column by adding a suitable eluting agent. Note that antibody that can be purified by the adsorbent of the present invention is an antibody that includes at least an Fc region having affinity for the FcyRIIa protein. Examples of the antibody used in antibody drugs include commonly used chimeric antibodies, humanized antibodies, human antibodies and amino acid substitution products thereof. Further, antibodies that have artificially been structurally modified, such as bispecific antibodies, fusion antibodies consisting of an antibody Fc region and another protein, or conjugates consisting of an antibody Fc region and a drug (antibody-drug conjugates, ADC) can also be purified with the adsorbent of the present invention. Furthermore, prior to adding the buffer containing the antibody into the column, by equilibrating the column using a suitable buffer solution, the antibodies can be purified to a higher degree of purity, which is preferable. Examples of the buffer solution include a phosphate buffer and other buffer solutions containing an inorganic salt as a component thereof. The pH thereof is pH 3.0 to 10.0, preferably pH 5.0 to 8.0.

In order to elute the antibody adsorbed to the adsorbent of the present invention, interactions between the antibody and the ligand (FcyRIIa protein of the present invention) can be weakened. Specific examples thereof include changing pH with a buffer solution, using a counter peptide, changing the temperature, and changing the salt concentration. Specific examples of eluents for eluting antibodies adsorbed to the adsorbent include buffer solutions that are more acidic than the solution used when adsorbing antibodies to the adsorbent of the present invention. Examples of this type of buffer solution include a citrate buffer solution, glycine-hydrochloric acid buffer solution and acetate buffer solution capable of buffering in the acidic range. The pH of the buffer solution may be set within a range that does not impair the antibody function and is preferably pH 2.5 to 6.0, more preferably pH 3.0 to 5.0, and even more preferably pH 3.3 to 4.0.

### [ADVANTAGEOUS EFFECTS OF INVENTION]

The FcyRIIa protein of the present invention is a protein wherein an amino acid residue at a specific site in the extracellular region of wild type human FcyRIIa has been substituted with another amino acid. The FcyRIIa protein of the present invention has improved acid stability compared to wildtype human FcyRIIa. Accordingly, the FcyRIIa protein of the present invention is useful as a ligand of an adsorbent for separating antibodies (immunoglobulins).

### BRIEF DESCRIPTION OF DRAWINGS

FIG. 1 is a schematic diagram of the FcyRIIa. The numbers in the drawing indicate the positions in the amino acid sequence of SEQ ID NO: 1. In the drawing, S represents a signal sequence, EC represents an extracellular region, TM represents a transmembrane region and C represents an intercellular region.
FIG. 2 is a graph indicating the results of evaluation of acid resistivity of Fc binding proteins in which an amino acid at one site or amino acids at two sites have been substituted as compared to the Fc binding protein consisting of the amino acid sequence set forth in SEQ ID NO: 3 (control).

### EXAMPLES

The following examples are provided to describe the present invention in more detail .

### EXAMPLE 1 Introduction of mutations into Fc-binding protein and construction of library

In an expression vector pET-Am6 for expressing the Fc-binding protein consisting of the amino acid sequence set forth in SEQ ID NO: 3, random mutations were introduced into the section of the polynucleotide (SEQ ID NO: 4) encoding the Fc-binding protein by error prone PCR. Note that the Fc-binding protein consisting of the sequence set forth in SEQ ID NO: 3 was obtained by introducing amino acid substitutions at the six sites indicated below into an Fc-binding protein consisting of the sequence set forth in SEQ ID NO: 2 and comprising the wildtype human FcyRIIa extracellular domain.
Substitution of isoleucine to valine at position 68 of SEQ ID NO: 2 (position 73 in SEQ ID NO: 1).
Substitution of histidine to glutamine at position 80 of SEQ ID NO: 2 (position 85 in SEQ ID NO: 1).
Substitution of serine to threonine at position 84 of SEQ ID NO: 2 (position 89 in SEQ ID NO: 1).
Substitution of asparagine to threonine at position 90 of SEQ ID NO: 2 (position 95 in SEQ ID NO: 1).
Substitution of asparagine to serine at position 91 of SEQ ID NO: 2 (position 96 in SEQ ID NO: 1).
Substitution of histidine to arginine at position 125 of SEQ ID NO: 2 (position 130 in SEQ ID NO: 1).

(1) Error prone PCR was performed using the above pET-Am6 as template DNA. Error prone PCR was performed as follows using the primers set forth in SEQ ID NOs: 8 and 9. A reaction solution having the composition shown in Table 1 was prepared and then subjected to heat treatment for 2 minutes at 95 °C, the reaction solution was then subjected to 35 reaction cycles each involving a first step at 95 °C for 30 seconds, a second step at 60 °C for 30 seconds, and a third step at 72 °C for 90 seconds, before finally undergoing heat treatment for 7 minutes at 72 °C.

### [Table 1]

**Table 1**

| COMPOSITION | VOLUME |
|---|---|
| Template DNA (10 ng/pL) | 1 µL |
| 10 mM MnCl₂ | 4 µL |
| 10 mM dATP | 2 pL |
| 10 mM dGTP | 2 pL |
| 10 mM dCTP | 12 µL |
| 10 mM dTTP | 8 µL |
| Primer(SEQ ID NO: 8, 10 pmol/µL) | 4 µL |
| Primer(SEQ ID NO: 9, 10 pmol/µL) | 4 µL |
| 10×Ex Taq Buffer | 10 µL |
| 25 mM MgCl₂ | 12 µL |
| GoTaq polymerase(Promega Corporation) | 1 µL |
| H₂O | up to 100 µL |

(2) The PCR product obtained in (1) was purified, digested with the restriction enzymes NcoI and HindIII, and then ligated into the expression vector pETMalE (Japanese Unexamined Patent Publication (Kokai) No. 2011-206046) which had been digested with the same restriction enzymes beforehand.

(3) Following completion of the ligation reaction, the reaction solution was introduced into *E. coli* BL21 (DE3) by the heat shock method, the *E. coli* were then cultured on LB plate medium containing 50 µg/mL of kanamycin (for 18 hours at 37 °C) and the colonies that formed on the plate were used as a random mutant library.

### EXAMPLE 2 Screening of acid resistant Fc-binding protein

(1) The random mutant library produced in EXAMPLE 1 (transformants) was inoculated into 200 µL of a 2YT liquid culture medium (peptone: 16 g/L, yeast extract: 10 g/L, sodium chloride: 5 g/L) containing 50 µg/mL of kanamycin, and then shake cultured overnight at 30 °C using a 96-well deep well plate.
(2) After culturing, 5 µL of the culture solution was subcultured in 500 µL of a 2YT liquid culture medium containing 0.05 mM of IPTG (isopropyl-β-D-thiogalactopyranoside), 0.3% (w/v) glycine and 50 µg/mL of kanamycin, followed by additional shake culturing overnight at 20 °C using a 96-well deep well plate.
(3) After culturing, 20 µL of a culture supernatant containing each Fc-binding protein obtained by centrifugation was mixed with 80 µL of 0.1 M glycine hydrochloride buffer (pH 3.0), and left standing for 24 hours at 25 °C.
(4) The antibody binding activity of the Fc-binding protein when subjected to acid treatment of (3) and the antibody binding activity of the Fc-binding protein when not subjected to acid treatment of (3) were each measured according to the ELISA procedure indicated below and residual activity was calculated by dividing the antibody binding activity when the Fc-binding protein was subjected to acid treatment by the antibody binding activity when the Fc-binding protein was not subjected to acid treatment. **[0045]**
   (4-1) A preparation of gamma-globulin (The Chemo-Sero-Therapeutic Research Institute), which is a human antibody, was immobilized in wells of a 96-well microplate at 1 µg/well (for 18 hours at 4 °C). Following completion of immobilization, the reaction was blocked with 20 mM Tris-hydrochloric acid buffer (pH 7.4) containing 2% (w/v) skim milk (manufactured by BD) and 150 mM sodium chloride.
   (4-2) The microplate was washed with a washing buffer (20 mM Tris-HCl buffer (pH 7.4) containing 0.05% (w/v) Tween 20 and 150 mM NaCl). Then a solution containing the Fc-binding protein to be evaluated for antibody binding activity was added and the Fc-binding protein was allowed to react with the immobilized gamma-globulin (for 1 hour at 30 °C).
   (4-3) Following completion of the reaction, the microplate was washed with the above washing buffer and then an Anti-6His antibody (manufactured by Bethyl Laboratories) diluted to 100 ng/mL was added at 100 µL/well.
   (4-4) The reaction was performed for 1 hour at 30 °C and washed with the above washing buffer and then TMB Peroxidase Substrate (manufactured by KPL) was added at 50 µL/well. Coloring was interrupted by adding 1 M phosphoric acid at 50 µL/well, and optical absorbance at 450 nm was measured with a microplate reader (Tecan Trading).
(5) Approximately 700 strains of transformants were evaluated using the method of (4), and therefrom, transformants that expressed Fc-binding protein having improved acid stability compared to the Fc-binding protein consisting of the amino acid sequence set forth in SEQ ID NO: 3 were selected. The selected transformants were then cultured and expression vectors were prepared using the QIAprep Spin Miniprep Kit (Qiagen).
(6) The sequence of the polynucleotide region encoding the Fc-binding protein inserted into the obtained expression vector was subjected to a cycle sequence reaction using the Big Dye Terminator Cycle Sequencing FS Read Reaction Kit (PE Applied Biosystems) based on the chain termination method, followed by analysis of the nucleotide sequence thereof with a fullyautomated DNA sequencer-ABI Prism 3700 DNA Analyzer (PE Applied Biosystems) and the sites of the amino acid mutations were identified. Note that during this analysis, an oligonucleotide consisting of the sequence set forth in either SEQ ID NO: 8 or 9 was used as the sequence primer.

The positions of amino acid substitutions and the residual activities (%) after acid treatment of Fc-binding proteins expressed by transformants selected in (5) with respect to the Fc-binding protein (control) consisting of the amino acid sequence set forth in SEQ ID NO: 3 are summarized in FIG. 2. Compared to the control, Fc-binding proteins which have undergone the following substitutions were found to have improved acid stability: Pro42Gln (this represents a substitution of proline to glutamine at position 42 of SEQ ID NO: 2 (disclosed but not part of the invention, position 47 in SEQ ID NO: 1), this also applies to the following), Pro63Leu (disclosed but not part of the invention), Asn75Ser (disclosed but not part of the invention), Leu163His (disclosed but not part of the invention), Asp179Val (disclosed but not part of the invention), Leu190Ser, Ile 198V al (disclosed but not part of the invention), Ile45Thr (disclosed but not part of the invention), Gln69Leu (disclosed but not part of the invention), Pro83Leu (disclosed but not part of the invention), or Thr123Pro (disclosed but not part of the invention). Accordingly, including at least one of the amino acid substitutions at the 11 sites in the extracellular domain of human FcyRIIa was found to improve acid stability (acid resistance). Since residual activity was the highest for Leu190Ser thereamong, it was found that Fc-binding proteins having at least the Leu190Ser amino acid substitution in the extracellular domain of human FcyRIIa have particularly improved acid stability (acid resistance).

Among the Fc-binding proteins with improved acid stability obtained in the present example, the amino acid sequence of the Fc-binding protein having the Leu190Ser amino acid substitution is set forth in SEQ ID NO: 5 and the sequence of a polynucleotide encoding the protein is set forth in SEQ ID NO: 6. Note that in SEQ ID NO: 5, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute a polypeptide capable of binding to the Fc region (FcyRIIa-m7), the glycines (Gly) at position 202 and position 203 constitute a linker sequence, and the histidines from position 204 to position 209 constitute a tag sequence. Further, FcyRIIa-m7 is a polypeptide comprising amino acid residues from the glutamine at position 29 to the glutamine at position 201 of the amino acid sequence set forth in SEQ ID NO: 2, and having the following amino acid substitutions in the amino acid residues from position 29 to position 201: Ile68Val, His80Gln, Ser84Thr, Asn90Thr, Asn91Ser, His125Arg, and Leu190Ser.

### EXAMPLE 3 Introduction of further amino acid substitutions (1)

The amino acid substitutions involved in improving the acid stability of the Fc-binding protein found in EXAMPLE 2 were further introduced into FcyRIIa-m7 to further improve the acid stability. Introduction (accumulation) of amino acid substitutions was mainly carried out using PCR, and the four types of polypeptides shown in (a) to (d) below were prepared.
(a) a polypeptide (disclosed but not part of the invention, named FcyRIIa-m8A) wherein an Ile45Thr amino acid substitution was further introduced to FcyRIIa-m7,
(b) a polypeptide (named FcyRIIa-m8B) wherein a Gln69Leu amino acid substitution was further introduced to FcyRIIa-m7,
(c) a polypeptide (disclosed but not part of the invention, named FcyRIIa-m8C) wherein a Pro83Leu amino acid substitution was further introduced to FcyRIIa-m7,
(d) a polypeptide (named FcyRIIa-m8D) wherein a Thr123Pro amino acid substitution was further introduced to FcyRIIa-m7.

The method of preparing each Fc-binding protein is described in detail below.

### (a) FcyRIIa-m8A

Ile45Thr was selected from among the amino acid substitutions contributing to acid stability elucidated in EXAMPLE 2 and was accumulated in FcyRIIa-m8 to prepare FcyRIIa-m8A. Specifically, a mutation causing Ile45Thr was introduced to the polynucleotide encoding FcyRIIa-m7 obtained in EXAMPLE 2 to prepare FcyRIIa-m8A.
(a-1) The polynucleotide (SEQ ID NO: 6) encoding the Fc-binding protein comprising FcyRIIa-m7 obtained in EXAMPLE 2 was used as a template and PCR was performed thereon using oligonucleotides consisting of the sequences set forth in SEQ ID NO: 8 and SEQ ID NO: 27 as PCR primers. PCR was carried out by preparing a reaction solution having the composition shown in Table 2 and then subjecting the solution to heat treatment for 5 minutes at 98 °C, followed by 30 reaction cycles each involving a first step at 98 °C for 10 seconds, a second step at 55 °C for 5 seconds, and a third step at 72 °C for 1 minute, before finally undergoing heat treatment for 5 minutes at 72 °C. The amplified PCR product was subjected to agarose gel electrophoresis and purified from the gel using QIAquick Gel Extraction Kit (manufactured by Qiagen). The purified PCR product was named m8A-F.

### [Table 2]

**Table 2**

| COMPOSITION | VOLUME |
|---|---|
| Template DNA | 2 µL |
| 2.5 mM dNTPs | 2 µL |
| 10 µM Forward primer | 1 µL |
| 10 µM Reverse primer | 1 µL |
| 5 × PrimeSTAR Buffer( TAKARA BIO INC. ) | 4 µL |
| 2.5 U /mL PrimeSTAR HS( TAKARA BIO INC. ) | 0.5 µL |
| H₂O | up to 20 µL |

(a-2) Using the same polynucleotide as in (a-1) as a template, PCR and PCR product purification were performed using the same methods as in (a-1) other than using oligonucleotides consisting of the sequences set forth in SEQ ID NO: 26 and SEQ ID NO: 9 as PCR primers. The purified PCR product was named m8A-S.
(a-3) The two types of PCR product obtained in (a-1) and (a-2) (m8A-F and m8A-S) were mixed and a reaction solution comprising the components shown in Table 3 was prepared. PCR was carried out by subjecting the reaction solution to heat treatment for 5 minutes at 98 °C, followed by 5 reaction cycles each involving a first step at 98 °C for 10 seconds, a second step at 55 °C for 5 seconds, and a third step at 72 °C for 1 minute and a PCR product m8A-FL in which the m8A-F and 8A-S were linked was obtained.

### [Table 3]

**Table 3**

| COMPOSITION | VOLUME |
|---|---|
| PCR product | 1 µL |
| 2.5 mM dNTPs | 2 µL |
| 5× PrimeSTAR Buffer( TAKARA BIO INC. ) | 4 µL |
| 2.5 U/mL PrimeSTAR HS( TAKARA BIO INC. ) | 0.5 pL |
| H₂O | up to 20 µL |

(a-4) The PCR product m8A-FL obtained in (a-3) was used as a template, and PCR was performed using oligonucleotides consisting of the sequences set forth in SEQ ID NO: 8 and SEQ ID NO: 9 as PCR primers. PCR was carried out by preparing a reaction solution having the composition shown in Table 4 and then subjecting the solution to heat treatment for 5 minutes at 98 °C, followed by 30 reaction cycles each involving a first step at 98 °C for 10 seconds, a second step at 55 °C for 5 seconds, and a third step at 72 °C for 1 minute. Thereby a polynucleotide encoding FcyRIIa-m8A was prepared.

### [Table 4]

**Table 4**

| COMPOSITION | VOLUME |
|---|---|
| PCR product | 2 µL |
| 2.5 mM dNTPs | 4 µL |
| 10 µM Forward primer | 2 µL |
| 10 µM Reverse primer | 2 µL |
| 5×PrimeSTAR Buffer( TAKARA BIO INC. ) | 10 µL |
| 2.5 U/mL PrimeSTAR HS( TAKARA BIO INC. ) | 1 µL |
| H₂O | up to 50 µL |

(a-5) The polynucleotide obtained in (a-4) was purified, digested with the restriction enzymes NcoI and HindIII, and then ligated into the expression vector pETMalE (Japanese Unexamined Patent Publication (Kokai) No. 2011-206046) which had been digested with the restriction enzymes NcoI and HindIII beforehand. This was used to transform *E. coli* BL21 (DE3).
(a-6) The obtained transformants were cultured in LB culture medium to which 50 µg/mL of kanamycin had been added. By extracting plasmids from the harvested bacteria (transformants) the plasmid pET-m8A containing a polynucleotide encoding FcyRIIa-m8A which is a polynucleotide with an additional amino acid substitution at one site with respect to FcyRIIa-m7 was obtained.
(a-7) Sequence analysis of the nucleotide sequence of pET-m8 was performed using the same method as in EXAMPLE 2 (6).

The amino acid sequence of the FcyRIIa-m8a to which a signal sequence and a polyhistidine tag have been added is set forth in SEQ ID NO: 11 and the sequence of a polynucleotide encoding this FcyRIIa-m8a is set forth in SEQ ID NO: 10. Note that in SEQ ID NO: 11, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcyRIIa-m8A amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### (b) FcyRIIa-m8B

Gln69Leu was selected from among the amino acid substitutions contributing to improved acid stability elucidated in EXAMPLE 2 and was accumulated in FcyRIIa-m7 to prepare FcyRIIa-m8B. Specifically, a mutation causing Gln69Leu was introduced to the polynucleotide encoding FcyRIIa-m7 obtained in EXAMPLE 2 to prepare FcyRIIa-m8B.
(b-1) The polynucleotide (SEQ ID NO: 6) encoding the Fc-binding protein comprising FcyRIIa-m7 obtained in EXAMPLE 2 was used as a template, and PCR and PCR product purification were performed using the same methods as in (a-1) other than using oligonucleotides consisting of the sequences set forth in SEQ ID NO: 8 and SEQ ID NO: 29 as PCR primers. The purified PCR product was named m8B-F.
(b-2) Using the same polynucleotide as in (b-1) as a template, PCR and PCR product purification were performed using the same methods as in (a-1) other than using oligonucleotides consisting of the sequences set forth in SEQ ID NO: 28 and SEQ ID NO: 9 as PCR primers. The purified PCR product was named m8B-S.
(b-3) PCR was performed using the same method as in (a-3) other than using m8B-F obtained in (b-1) and m8B-S obtained in (b-2) as PCR products, and a PCR product m8B-FL having m8B-F and m8B-S linked together was obtained.
(b-4) The PCR product m8B-FL obtained in (b-3) was used as a template, and PCR was performed using the same method as in (a-4) other than using oligonucleotides consisting of the sequences set forth in SEQ ID NO: 8 and SEQ ID NO: 9 as PCR primers. Thereby, a polynucleotide encoding FcyRIIa-m8B was prepared.
(b-5) The polynucleotide obtained in (b-4) was purified, digested with the restriction enzymes NcoI and HindIII, and then ligated into the expression vector pETMalE (Japanese Unexamined Patent Publication (Kokai) No. 2011-206046) which had been digested with restriction enzymes NcoI and HindIII beforehand. This was used to transform *E*. *coli* BL21 (DE3).
(b-6) The obtained transformants were cultured in LB culture medium to which 50 µg/mL of kanamycin had been added. By extracting plasmids from the harvested bacteria (transformants) the plasmid pET-FcyRIIa-m8B containing a polynucleotide encoding FcyRIIa-m8B which is a polynucleotide with an additional amino acid substitution at one site with respect to FcyRIIa-m7 was obtained.
(b-7) Sequence analysis of the nucleotide sequence of pET-FcyRIIa-m8 was performed using the same method as in EXAMPLE 2 (6).

The amino acid sequence of the FcyRIIa-m8B to which a signal sequence and a polyhistidine tag have been added is set forth in SEQ ID NO: 13 and the polynucleotide sequence encoding this FcyRIIa-m8a is set forth in SEQ ID NO: 12. Note that in SEQ ID NO: 13, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcyRIIa-m8B amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### (c) FcyRIIa-m8C

Pro83Leu was selected from among the amino acid substitutions contributing to improved acid stability elucidated in EXAMPLE 2 and was accumulated in FcyRIIa-m7 to prepare FcyRIIa-m8C. Specifically, a mutation causing Pro83Leu was introduced to the polynucleotide encoding FcyRIIa-m7 obtained in EXAMPLE 2 to prepare FcyRIIa-m8C.
(c-1) The polynucleotide (SEQ ID NO: 6) encoding the Fc-binding protein comprising FcyRIIa-m7 obtained in EXAMPLE 2 was used as a template, and PCR and PCR product purification were performed using the same methods as in (a-1) other than using oligonucleotides consisting of the sequences set forth in SEQ ID NO: 8 and SEQ ID NO: 31 as PCR primers. The purified PCR product was named m8C-F.
(c-2) Using the same polynucleotide as in (c-1) as a template, PCR and PCR product purification was performed using the same method as in (a-1) other than using oligonucleotides consisting of the sequences set forth in SEQ ID NO: 30 and SEQ ID NO: 9 as PCR primers. The purified PCR product was named m8C-S.
(c-3) PCR was performed using the same method as in (a-3) other than using m8C-F obtained in (c-1) and m8C-S obtained in (c-2) as PCR products, and a PCR product m8C-FL having m8C-F and m8C-S linked together was obtained.
(c-4) The PCR product m8C-FL obtained in (c-3) was used as a template, and PCR was performed using the same method as in (a-4) other than using oligonucleotides consisting of the sequences set forth in SEQ ID NO: 8 and SEQ ID NO: 9 as PCR primers. Thereby, a polynucleotide encoding FcyRIIa-m8C was prepared.
(c-5) The polynucleotide obtained in (c-4) was purified, digested with the restriction enzymes NcoI and HindIII, and then ligated into the expression vector pETMalE (Japanese Unexamined Patent Publication (Kokai) No. 2011-206046) which had been digested with the restriction enzymes NcoI and HindIII beforehand. This was used to transform *E. coli* BL21 (DE3).
(c-6) The obtained transformants were cultured in LB culture medium to which 50 µg/mL of kanamycin had been added. By extracting plasmids from the harvested bacteria (transformants) the plasmid pET-FcyRIIa-m8C containing a polynucleotide encoding FcyRIIa-m8C which is a polynucleotide with an additional amino acid substitution at one site with respect to FcyRIIa-m7 was obtained.
(c-7) Sequence analysis of the nucleotide sequence of pET-FcyRIIa-m8C was performed using the same method as in EXAMPLE 2 (6).

The amino acid sequence of the FcyRIIa-m8C to which a signal sequence and a polyhistidine tag have been added is set forth in SEQ ID NO: 15 and the polynucleotide sequence encoding this FcyRIIa-m8C is set forth in SEQ ID NO: 14. Note that in SEQ ID NO: 15, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcyRIIa-m8C amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### (d) FcyRIIa-m8D

Thr123Pro was selected from among the amino acid substitutions contributing to improved acid stability elucidated in EXAMPLE 2 and was accumulated in FcyRIIa-m7 to prepare FcyRIIa-m8D. Specifically, a mutation causing Thr123Pro was introduced to the polynucleotide encoding FcyRIIa-m7 obtained in EXAMPLE 2 to prepare FcyRIIa-m8D.
(d-1) The polynucleotide (SEQ ID NO: 6) encoding the Fc-binding protein comprising FcyRIIa-m7 obtained in EXAMPLE 2 was used as a template, and PCR and PCR product purification were performed using the same methods as in (a-1) other than using oligonucleotides consisting of the sequences set forth in SEQ ID NO: 8 and SEQ ID NO: 33 as PCR primers. The purified PCR product was named m8D-F.
(d-2) Using the same polynucleotides as in (d-1) as a template, PCR and PCR product purification were performed using the same methods as in (a-1) other than using oligonucleotides consisting of the sequences set forth in SEQ ID NO: 32 and SEQ ID NO: 9 as PCR primers. The purified PCR product was named m8D-S.
(d-3) PCR was performed using the same method as in (a-3) other than using m8C-F obtained in (d-1) and m8C-S obtained in (d-2) as PCR products, and a PCR product m8D-FL having m8D-F and m8D-S linked together was obtained.
(d-4) The PCR product m8D-FL obtained in (d-3) was used as a template, and PCR was performed using the same method as in (a-4) other than using oligonucleotides consisting of the sequences set forth in SEQ ID NO: 8 and SEQ ID NO: 9 as PCR primers. Thereby, a polynucleotide encoding FcyRIIa-m8D was prepared.
(d-5) The polynucleotide obtained in (d-4) was purified, digested with the restriction enzymes NcoI and HindIII, and then ligated into the expression vector pETMalE (Japanese Unexamined Patent Publication (Kokai) No. 2011-206046) which had been digested with the restriction enzymes NcoI and HindIII beforehand. This was used to transform *E. coli* BL21 (DE3).
(d-6) The obtained transformants were cultured in LB culture medium to which 50 µg/mL of kanamycin had been added. By extracting plasmids from the harvested bacteria (transformants) the plasmid pET-FcyRIIa-mD8 containing a polynucleotide encoding FcyRIIa-mD8 which is a polynucleotide with an additional amino acid substitution at one site with respect to FcyRIIa-m7 was obtained.
(d-7) Sequence analysis of the nucleotide sequence of pET-FcyRIIa-mD8 was performed using the same method as in EXAMPLE 2 (6).

The amino acid sequence of the FcyRIIa-m8D to which a signal sequence and a polyhistidine tag have been added is set forth in SEQ ID NO: 17 and the polynucleotide sequence encoding this FcyRII-m8D is set forth in SEQ ID NO: 16. Note that in SEQ ID NO: 17, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcyRIIa-m8D amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### EXAMPLE 4 Evaluation of acid stability of Fc-binding protein (1)

(1) The transformants for expressing FcyRII-m6 (disclosed but not part of the invention), as well FcyRII-m8A disclosed but not part of the invention,, FcyRII-m8B, FcyRII-m8C disclosed but not part of the invention" and FcyRII-m8D prepared in EXAMPLE 3 were each precultured by inoculation into 3 mL of 2YT liquid medium (peptone: 16 g/L, yeast extract: 10 g/L, sodium chloride: 5 g/L) containing 50 µg/mL of kanamycin and then aerobic shake culturing overnight at 37 °C.
(2) 200 µL of the preculture solution was inoculated into 20 mL of 2YT liquid medium to which 50 µg/mL of kanamycin had been added, then aerobically shake cultured at 37 °C.
(3) 1.5 hours after culturing had been started, the culturing temperature was changed to 20 °C followed by shake culturing for 30 minutes. IPTG was then added to a final concentration of 0.01 mM, then shake culturing was continued aerobically overnight at 20 °C.
(4) Following completion of culturing, the bacterial cells were harvested by centrifugation to prepare a protein extract solution using the BugBuster Protein Extraction Kit (Takara Bio).
(5) The antibody binding activities of FcγRIIa-m6, FcyRIIa-m8A, FcyRIIa-m8B, FcyRIIa-m8C, and FcyRIIa-m8D in the protein extract prepared in (4) were measured using the ELISA method described in (4) of EXAMPLE 2. At this time, protein concentrations were measured by preparing a calibration curve using the purified and quantified FcyRIIa-m6.
(6) Each of the Fc-binding proteins was diluted to a concentration of 10 µg/mL with pure water, then acid treatment was performed thereon by mixing 50 µL of the above diluted solutions and 50 µL of 20 mM glycine-hydrochloric acid buffer solution (pH 3.0) and leaving the mixture to stand for 24 hours at 30 °C. Thereafter, the mixture was neutralized with a four-fold dilution with 1 M Tris-hydrochloric acid buffer solution (pH 7.0) and the binding activity of the Fc-binding protein was measured using the ELISA method described in EXAMPLE 2 (4).
(7) The residual activity was calculated by dividing the antibody binding activity when acid treatment was performed by the antibody binding activity when the acid treatment was not performed, and acid stability was evaluated.

The results are illustrated in Table 5. Among the Fc-binding proteins prepared in EXAMPLE 3, since FcyRIIa-m8B and FcyRIIa-m8D had a higher residual activity than FcyRIIa-m6, it was confirmed that the acid stability of FcyRIIa-m8B and FcyRIIa-m8D had been improved compared to FcyRIIa-m6.

### [Table 5]

**Table 5**

| EXAMPLE | NAME | SEQ ID NO | RESIDUAL ACTIVITY [%] |
|---|---|---|---|
| EXAMPLE 3(a) | FcγRIIa-m8A | 11 | 55.7 |
| EXAMPLE 3(b) | FcγRIIa-m8B | 13 | 73.5 |
| EXAMPLE 3(c) | FcγRIIa-m8C | 15 | 25.2 |
| EXAMPLE 3(d) | FcγRIIa-mBD | 17 | 73.8 |
| | FcγRIIa-m6 | 3 | 56.9 |

### EXAMPLE 5 Introduction of further amino acid substitutions (2)

The amino acid substitutions involved in improving the acid stability of the Fc-binding protein found in EXAMPLE 2 were further introduced into FcyRIIa-m8D to further improve the acid stability. Introduction (accumulation) of amino acid substitutions was mainly carried out using PCR and the three types of polypeptides shown in (a) to (c) below were prepared.
(a) a polypeptide (named FcγRIIa-m10A) wherein an Ile45Thr and a Gln69Leu acid amino substitution were further introduced to FcyRIIa-m8D,
(b) a polypeptide (disclosed but not part of the invention, named FcγRIIa-m10B) wherein an Ile45Thr and a Pro83Leu amino acid substitution were further introduced to FcyRIIa-m8D,
(c) a polypeptide (named FcγRIIa-m10C) wherein a Gln69Leu and a Pro83Leu amino acid substitution were further introduced to FcyRIIa-m8D.

The method for preparing each Fc-binding protein is described in detail below.

### (a) FcγRIIa-m10A

Ile45Thr and Gln69Leu were selected from among the amino acid substitutions contributing to improved acid stability elucidated in EXAMPLE 2 and were accumulated in FcyRIIa-m8D to prepare FcγRIIa-m10A. Specifically, mutations causing Ile45Thr and Gln69Leu were introduced to the polynucleotide encoding FcyRIIa-m8D obtained in EXAMPLE 3 (d) to prepare FcγRIIa-m10A.

(a-1) The polynucleotide (SEQ ID NO: 16) encoding the Fc-binding protein comprising FcyRIIa-m8D obtained in EXAMPLE 3 (d) was used as a template, and PCR and PCR product purification were performed using the same methods as in EXAMPLE 3 (a-1) other than using oligonucleotides consisting of the sequences set forth in SEQ ID NO: 8 and SEQ ID NO: 27 as PCR primers. The purified PCR product was named m10A-F.
(a-2) Using the same polynucleotide as in (a-1) as a template, PCR and PCR product purification were performed using the same methods as in EXAMPLE 3 (a-1) other than using oligonucleotides consisting of the sequences set forth in SEQ ID NO: 26 and SEQ ID NO: 29 as PCR primers. The purified PCR product was named m10A-S.
(a-3) Using the same polynucleotide as in (a-1) as a template, PCR and PCR product purification were performed using the same methods as in EXAMPLE 3 (a-1) other than using oligonucleotides consisting of the sequences set forth in SEQ ID NO: 28 and SEQ ID NO: 9 as PCR primers. The purified PCR product was named m10A-T.
(a-4) PCR was performed using the same method as in EXAMPLE 3 (a-3), other than using m10A-F obtained in (a-1), m10A-S obtained in (a-2), and m10A-T obtained in (a-3) as PCR products, and the PCR product m10-FL having m10A-F, m10A-S, and m10A-T linked together was obtained.
(a-5) The PCR product m10A-FL obtained in (a-4) was used as a template, and PCR was performed using the same method as in EXAMPLE 3 (a-4) other than using oligonucleotides consisting of the sequences set forth in SEQ ID NO: 8 and SEQ ID NO: 9 as PCR primers. Thereby, a polynucleotide encoding FcγRIIa-m10A was prepared.
(a-6) The polynucleotide obtained in (a-5) was purified, digested with the restriction enzymes NcoI and HindIII, and then ligated into the expression vector pETMalE (Japanese Unexamined Patent Publication (Kokai) No. 2011-206046) which had been digested with NcoI and HindIII beforehand. This was used to transform *E*. *coli* BL21 (DE3).
(a-7) The obtained transformants were cultured in LB culture medium to which 50 µg/mL of kanamycin had been added. By extracting plasmids from the harvested bacteria (transformants) the plasmid pET-FcγRIIa-m10A containing a polynucleotide encoding FcγRIIa-m10A which is a polynucleotide with additional amino acid substitutions at three sites with respect to FcyRIIa-m7 was obtained.
(a-8) Sequence analysis of the nucleotide sequence of pET-FcγRIIa-m10A was performed using the same method as in EXAMPLE 2 (6).

The amino acid sequence of FcγRIIa-m10A to which a signal sequence and a polyhistidine tag have been added is set forth in SEQ ID NO: 19 and the polynucleotide sequence encoding this FcγRIIa-m10A is set forth in SEQ ID NO: 18. Note that in SEQ ID NO: 19, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcγRIIa-m10A amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### (b) FcγRIIa-m10B

Ile45Thr and Pro83Leu were selected from among the amino acid substitutions contributing to improved acid stability elucidated in EXAMPLE 2 and were accumulated in FcyRIIa-m8D to prepare FcγRIIa-m10B. Specifically, mutations causing Ile45Thr and Pro83Leu were introduced to the polynucleotide encoding FcyRIIa-m8D obtained in EXAMPLE 3(d) to prepare FcγRIIa-m10B.
(b-1) The polynucleotide (SEQ ID NO: 16) encoding the Fc-binding protein comprising FcyRIIa-m8D obtained in EXAMPLE 3(d) was used as a template, and PCR and PCR product purification were performed using the same methods as in EXAMPLE 3 (a-1) other than using oligonucleotides consisting of the sequences set forth in SEQ ID NO: 8 and SEQ ID NO: 27 as PCR primers. The purified PCR product was named m10B-F.
(b-2) Using the same polynucleotide as in (b-1) as a template, PCR and PCR product purification were performed using the same methods as in EXAMPLE 3 (a-1) other than using oligonucleotides consisting of the sequences set forth in SEQ ID NO: 26 and SEQ ID NO: 31 as PCR primers. The purified PCR product was named m10B-S.
(b-3) Using the same polynucleotide as in (b-2) as a template, PCR and PCR product purification were performed using the same methods as in EXAMPLE 3 (a-1) other than using oligonucleotides consisting of the sequences set forth in SEQ ID NO: 30 and SEQ ID NO: 9 as PCR primers. The purified PCR product was named m10B-T.
(b-4) PCR was performed using the same method as in EXAMPLE 3 section (a-3) other than using m10B-F obtained in (b-1), m10B-S obtained in (b-2), and m10B-T obtained in (b-3) as PCR products, and the PCR product m10B-FL having m10B-F, m10B-S, and m10B-T linked together was obtained.
(b-5) The PCR product m10B-FL obtained in (b-4) was used as a template, and PCR was performed using the same method as in EXAMPLE 3 section (a-4) other than using oligonucleotides consisting of the sequences set forth in SEQ ID NO: 8 and SEQ ID NO: 9 as PCR primers. Thereby, a polynucleotide encoding FcγRIIa-m10B was prepared.
(b-6) The polynucleotide obtained in (b-5) was purified, digested with the restriction enzymes NcoI and HindIII, and then ligated into the expression vector pETMalE (Japanese Unexamined Patent Publication (Kokai) No. 2011-206046) which had been digested with NcoI and HindIII beforehand. This was used to transform *E*. *coli* BL21 (DE3).
(b-7) The obtained transformants were cultured in LB culture medium to which 50 µg/mL of kanamycin had been added. By extracting plasmids from the harvested bacteria (transformants) the plasmid pET-FcγRIIa-m10B containing a polynucleotide encoding FcγRIIa-m10B which is a polypeptide with additional amino acid substitutions at three sites with respect to FcyRIIa-m7 was obtained.
(b-8) Sequence analysis of the nucleotide sequence of pET-FcγRIIa-m10B was performed using the same method as in EXAMPLE 2 (6).

The amino acid sequence of the FcγRIIa-m10B to which a signal sequence and a polyhistidine tag have been added is set forth in SEQ ID NO: 21 and the polynucleotide sequence encoding this FcγRIIa-m10B is set forth in SEQ ID NO: 20. Note that in SEQ ID NO: 21, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcγRIIa-m10B amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### (c) FcγRIIa-m10C

Gln69Leu and Pro83Leu were selected from among the amino acid substitutions contributing to improved acid stability elucidated in EXAMPLE 2 and were accumulated in FcyRIIa-m8D to prepare FcγRIIa-m10C. Specifically, mutations causing Gln69Leu and Pro83Leu were introduced to the polynucleotide encoding FcyRIIa-m8D obtained in EXAMPLE 3 (d) to prepare FcγRIIa-m10C.
(c-1) The polynucleotide (SEQ ID NO: 16) encoding the Fc-binding protein comprising FcyRIIa-m8D obtained in EXAMPLE 3(d) was used as a template, and PCR and PCR product purification were performed using the same methods as in EXAMPLE 3 (a-1) other than using oligonucleotides consisting of the sequences set forth in SEQ ID NO: 8 and SEQ ID NO: 29 as PCR primers. The purified PCR product was named m10C-F.
(c-2) Using the same polynucleotide as in (c-1) as a template, PCR and PCR product purification were performed using the same methods as in EXAMPLE 3 (a-1) other than using oligonucleotides consisting of the sequences set forth in SEQ ID NO: 28 and SEQ ID NO: 31 as PCR primers. The purified PCR product was named m10C-S.
(c-3) Using the same polynucleotide as in (c-1) as a template, PCR and PCR product purification was performed with the same method as in EXAMPLE 3 (a-1) other than using oligonucleotides consisting of the sequences set forth in SEQ ID NO: 30 and SEQ ID NO: 9 as PCR primers. The purified PCR product was named m10C-T.
(c-4) PCR was performed using the same method as in EXAMPLE 3 section (a-3), other than using m10C-F obtained in (c-1), m10C-S obtained in (c-2), and m10C-T obtained in (c-3) as PCR products, and the PCR product m10C-FL having m10C-F, m10C-S, and m10C-T linked together was obtained.
(c-5) The PCR product m10C-FL obtained in (c-4) was used as a template, and PCR was performed using the same method as in EXAMPLE 3 section (a-4) other than using oligonucleotides consisting of the sequences set forth in SEQ ID NO: 8 and SEQ ID NO: 9 as PCR primers. Thereby, a polynucleotide encoding FcγRIIa-m10C was prepared.
(c-6) The polynucleotide obtained in (c-5) was purified, digested with the restriction enzymes NcoI and HindIII, and then ligated into the expression vector pETMalE (Japanese Unexamined Patent Publication (Kokai) No. 2011-206046) which had been digested with the restriction enzymes NcoI and HindIII beforehand. This was used to transform *E. coli* BL21 (DE3).
(c-7) The obtained transformants were cultured in LB culture medium to which 50 µg/mL of kanamycin had been added. By extracting plasmids from the harvested bacteria (transformants) the plasmid pET-FcγRIIa-m10C containing a polynucleotide encoding FcγRIIa-m10C which is a polypeptide with additional amino acid substitutions at three sites with respect to FcyRIIa-m7 was obtained.
(c-8) Sequence analysis of the nucleotide sequence of pET-FcγRIIa-m10C was performed using the same method as in EXAMPLE 2 (6).

The amino acid sequence of the FcγRIIa-m10C to which a signal sequence and a polyhistidine tag have been added is set forth in SEQ ID NO: 23 and the polynucleotide sequence encoding this FcγRIIa-m10C is set forth in SEQ ID NO: 22. Note that in SEQ ID NO: 23, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcγRIIa-m10C amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### EXAMPLE 6 Introduction of further amino acid substitutions (3)

Ile45Thr, Gln69Leu, and Pro83Leu were selected from among the amino acid substitutions involved in improving the acid stability of the Fc-binding protein elucidated in EXAMPLE 2, and further introduced into FcyRIIa-m8D to further improve the acid stability. Specifically, a mutation causing Pro83Leu was introduced to the polynucleotide encoding FcγRIIa-m10A obtained in EXAMPLE 5 (a) to prepare a protein (named FcγRIIa-m11). The method of preparing FcγRIIa-m11 is described in detail below.

(1) The polynucleotide (SEQ ID NO: 18) encoding the Fc-binding protein comprising FcγRIIa-m10A obtained in EXAMPLE 5(a) was used as a template, and PCR and PCR product purification were performed using the same methods as in EXAMPLE 3(a-1) other than using oligonucleotides consisting of the sequences set forth in SEQ ID NO: 8 and SEQ ID NO: 31 as PCR primers. The purified PCR product was named m11-F.
(2) Using the same polynucleotide as in (1) as a template, PCR and PCR product purification were performed using the same methods as in EXAMPLE 3 (a-1) other than using oligonucleotides consisting of the sequences set forth in SEQ ID NO: 30 and SEQ ID NO: 9 as PCR primers. The purified PCR product was named m11-S
(3) PCR was performed using the same method as in EXAMPLE 3 section (a-3) other than using m11-F obtained in (1) and m11-S obtained in (2) as PCR products, and the PCR product m11-FL having m11-F and m11-S linked together was obtained.
(4) The PCR product m11-FL obtained in (3) was used as a template, and PCR was performed using the same method as in EXAMPLE 3 section (a-4) other than using oligonucleotides consisting of the sequences set forth in SEQ ID NO: 8 and SEQ ID NO: 9 as PCR primers. Thereby, a polynucleotide encoding FcγRIIa-m11 was prepared.
(5) The polynucleotide obtained in (4) was purified, digested with the restriction enzymes NcoI and HindIII, and then ligated into the expression vector pETMalE (Japanese Unexamined Patent Publication (Kokai) No. 2011-206046) which had been digested with the restriction enzymes NcoI and HindIII beforehand. This was used to transform *E. coli* BL21 (DE3).
(6) The obtained transformants were cultured in LB culture medium to which 50 µg/mL of kanamycin had been added. By extracting plasmids from the harvested bacteria (transformants) the plasmid pET-FcγRIIa-m11 containing a polynucleotide encoding FcyRIIa-m11 which is a polynucleotide with additional amino acid substitutions at four sites with respect to FcyRIIa-m7 was obtained.
(7) Sequence analysis of the nucleotide sequence of pET-FcγRIIa-m11 was performed using the same method as in EXAMPLE 2 (6).

The amino acid sequence of the FcγRIIa-m11 to which a signal sequence and a polyhistidine tag have been added is set forth in SEQ ID NO: 25 and the polynucleotide sequence encoding this FcγRIIa-m11 is set forth in SEQ ID NO: 24. Note that in SEQ ID NO: 25, the methionine (Met) at position 1 to the alanine (Ala) at position 26 constitute a MalE signal peptide, the methionine (Met) at position 27 and the glycine (Gly) at position 28 constitute a linker sequence, the glutamine (Gln) at position 29 to the glutamine (Gln) at position 201 constitute the FcγRIIa-m11 amino acid sequence (corresponding to the region of SEQ ID NO: 1 from position 34 to position 206), the glycines (Gly) at position 202 and position 203 constitute a linker sequence, and the histidines (His) from position 204 to position 209 constitute a tag sequence.

### EXAMPLE 7 Evaluation of acid stability of Fc-binding protein (2)

(1) The transformants for expressing FcyRIIa-m6 (disclosed but not part of the invention), as well as FcγRIIa-m10A, FcγRIIa-m10B (disclosed but not part of the invention), and FcγRIIa-m10C prepared in EXAMPLE 5, in addition to FcγRIIa-m11 prepared in EXAMPLE 6, were cultured and the proteins prepared by extraction therefrom using the same method as in EXAMPLE 4 (1) to (4).
(2) The antibody binding activities of FcyRIIa-m6, the three types of FcyRIIa-m10, and FcγRIIa-m11 in the protein extract solution prepared in (1) were measured using the ELISA method described in Example 2 (4). At this time, protein concentrations were measured by preparing a calibration curve using the purified and quantified FcyRIIa-m6.
(3) Each of the Fc-binding proteins was diluted to a concentration of 10 µg/mL with pure water, then acid treatment was performed thereon by mixing 50 µL of the above diluted solution and 50 µL of 20 mM glycine-hydrochloric acid buffer solution (pH 3.0), and leaving the mixture to stand for 24 hours at 30 °C. Thereafter, the mixture was neutralized with a four-fold dilution with 1 M Tris-hydrochloric acid buffer solution (pH 7.0) and the antibody binding activity of the Fc-binding protein was measured using the ELISA method described in Example 2 (4).
(4) The residual activity was calculated by dividing the antibody binding activity when acid treatment was performed by the antibody binding activity when the acid treatment was not performed, and acid stability was evaluated.

The results are illustrated in Table 6. Since FcγRIIa-m10A, FcγRIIa-m10C, and FcyRIIa-m11 had a higher residual activity than FcyRIIa-m6, it was confirmed that the acid stability of these proteins had been improved compared to FcyRIIa-m6. Thereamong, since FcγRIIa-m11 had a high residual activity of 95.3%, FcγRIIa-m11 was considered to be an Fc binding protein having particularly improved acid stability.

**[Table 6]**

| EXAMPLE | NAME | SEQ ID NO | RESIDUAL ACTIVITY [%] |
|---|---|---|---|
| EXAMPLE 5(a) | FcγRIIa-m10A | 19 | 77.3 |
| EXAMPLE 5(b) | FcγRIIa-m10B | 21 | 46.9 |
| EXAMPLE 5(c) | FcγRIIa-m10C | 23 | 72.9 |
| EXAMPLE 6 | FcγRIIa-m11 | 25 | 95.3 |
| | FcγRIIa-m6 | 3 | 56.9 |

### EXAMPLE 8 Deletion of amino acid residues in loop region

In order to further improve acid stability, amino acid residues positioned in the loop region of FcyRIIa were deleted on the basis of knowledge of crystal structure data of FcyRIIa registered in public databases (PDB ID: 1FCG). Specifically, some or all amino acid residues in the loop region (the glutamine at position 58 to the serine at position 67 of SEQ ID NO: 25) were deleted from the FcγRIIa-m11 protein obtained in EXAMPLE 6 to prepare the Fc-binding proteins indicated in (a) to (j) below by the following method.
a) an Fc-binding protein wherein the serine at position 62 of FcγRIIa-m11 was deleted (named FcγRII-m11-Del1)
b) an Fc-binding protein wherein the serine at position 62 and the glutamic acid in position 64 of FcγRIIa-m11 were deleted (disclosed but not part of the invention, named FcγRII-m11-Del2)
c) an Fc-binding protein wherein the amino acid residues from the serine at position 62 to the glutamic acid in position 64 of FcγRIIa-m11 were deleted (disclosed but not part of the invention, named FcγRII-m11-Del3),
d) an Fc-binding protein wherein the amino acid residues from the arginine at position 61 to the glutamic acid at position 64 of FcγRIIa-m11 were deleted (disclosed but not part of the invention, named FcγRII-m11-Del4),
e) an Fc-binding protein wherein the amino acid residues from the arginine at position 61 to the serine at position 65 of FcγRIIa-m11 were deleted (named FcγRII-m11-Del5),
f) an Fc-binding protein wherein the amino acid residues from the arginine at position 60 to the serine at position 65 of FcγRIIa-m11 were deleted (disclosed but not part of the invention, named FcγRII-m11-Del6),
g) an Fc-binding protein wherein the amino acid residues from the glycine at position 59 to the serine at position 65 of FcγRIIa-m11 were deleted (disclosed but not part of the invention, named FcγRII-m11-Del7),
h) an Fc-binding protein wherein the amino acid residues from the glycine at position 59 to the aspartic acid at position 66 of FcγRIIa-m11 were deleted (disclosed but not part of the invention, named FcγRII-m11-Del8),
i) an Fc-binding protein wherein the amino acid residues from the glycine at position 59 to the serine at position 67 of FcγRIIa-m11 were deleted (disclosed but not part of the invention, named FcγRII-m11-Del9),
j) an Fc-binding protein wherein the amino acid residues from the glutamine at position 58 to the serine at position 67 of FcγRIIa-m11 were deleted (disclosed but not part of the invention, named FcγRII-m11-Del10).
   (1) Polypeptides comprising the above 10 types of Fc-binding protein deletion ((a) FcγRII-m11-Del1: SEQ ID NO: 34, (b) FcγRII-m11-Del2: SEQ ID NO: 36, (c) FcγRII-m11-Del3: SEQ ID NO: 38, (d) FcγRIIa-m11-Del4: SEQ ID NO: 40, (e) FcγRII-m11-Del5: SEQ ID NO: 42, (f) FcγRII-m11-Del6: SEQ ID NO: 44, (g) FcγRII-m11-Del7: SEQ ID NO: 46, (h) FcγRII-m11-Del8: SEQ ID NO: 48, (i) FcγRII-m11-Del9: SEQ ID NO: 50, and (j) FcγRII-m11-Del10: SEQ ID NO: 52) were prepared by artificial gene synthesis (carried out by FASMAC).
   (2) The polynucleotides prepared in (1) were ligated into the expression vector pETMalE (Japanese Unexamined Patent Publication (Kokai) No. 2011-206046) which had been digested with the restriction enzymes NcoI and HindIII. These were used to transform *E. coli* BL21 (DE3).
   (3) The resulting transformants were cultured and the proteins prepared by extraction therefrom using the same method as in EXAMPLE 4 (1) to (4).

The amino acid sequence of (a) FcγRIIa-m11-Del1 to which a signal sequence and a polyhistidine tag have been added is set forth in SEQ ID NO: 35, the amino acid sequence of (b) FcγRIIa-m11-Del2 (disclosed but not part of the invention) to which a signal sequence and a polyhistidine tag have been added is set forth in SEQ ID NO: 37, the amino acid sequence of (c) FcγRIIa-m11-Del3 (disclosed but not part of the invention) to which a signal sequence and a polyhistidine tag has been added is set forth in SEQ ID NO: 39, the amino acid sequence of (d) FcγRIIa-m11-Del4 (disclosed but not part of the invention) to which a signal sequence and a polyhistidine tag have been added is set forth in SEQ ID NO: 41, the amino acid sequence of (e) FcγRIIa-m11-Del5 to which a signal sequence and a polyhistidine tag have been added is set forth in SEQ ID NO: 43, the amino acid sequence of (f) FcγRIIa-m11-Del6 (disclosed but not part of the invention) to which a signal sequence and a polyhistidine tag have been added is set forth in SEQ ID NO: 45, the amino acid sequence of (g) FcγRIIa-m11-Del7 (disclosed but not part of the invention) to which a signal sequence and a polyhistidine tag have been added is set forth in SEQ ID NO: 47, the amino acid sequence of (h) FcyRIIa-m11-Del 8 (disclosed but not part of the invention) to which a signal sequence and a polyhistidine tag have been added is set forth in SEQ ID NO: 49, the amino acid sequence of (i) FcγRIIa-m11-Del9 (disclosed but not part of the invention) to which a signal sequence and a polyhistidine tag have been added is set forth in SEQ ID NO: 51, the amino acid sequence of (j) FcγRIIa-m11-Del10 (disclosed but not part of the invention) to which a signal sequence and a polyhistidine tag have been added is set forth in SEQ ID NO: 53. Note that in these sequences, the methionine (Met) at position 1 to the alanine (Ala) at position 26 constitute a MalE signal peptide, the methionine (Met) at position 27 and the glycine (Gly) at position 28 constitute a linker sequence, the six histidine (His) residues at the C-terminal side constitute a tag sequence, the glycines (Gly) at position 7 and position 8 from the C-terminal side constitute a linker sequence, other regions constitute polypeptides capable of binding to the Fc region (FcγRIIa-m11-Del1, FcγRIIa-m11-Del2, FcγRIIa-m11-Del3, FcγRIIa-m11-Del4, FcγRIIa-m11-Del5, FcγRIIa-m11-Del6, FcγRIIa-m11-Del7, FcγRIIa-m11-Del8, FcγRIIa-m11-Del9, or FcγRIIa-m11-Del10).

### EXAMPLE 9 Evaluation of acid stability of Fc-binding protein (3)

(1) Transformants for expressing FcγRIIa-m11 prepared in EXAMPLE 6 were cultured and the proteins prepared by extraction therefrom using the same method as in EXAMPLE 4(1) to (4).
(2) The antibody binding activities in the protein extract solution prepared in (1) and EXAMPLE 8 were measured using the ELISA method described in Example 2 (4). At this time, protein concentrations were measured by preparing a calibration curve using the purified and quantified FcyRIIa-m6. Note that the protein concentrations of FcγRIIa-m11-Del8 (disclosed but not part of the invention), FcγRIIa-m11-Del9 (disclosed but not part of the invention), and FcγRIIa-m11-Del 10 (disclosed but not part of the invention) could not be measured due to insufficient protein expression.
(3) Each of the Fc-binding proteins was diluted to a concentration of 10 µg/mL with pure water, then acid treatment was performed thereon by mixing 50 µL of the diluted solution and 50 µL of 20 mM glycine-hydrochloric acid buffer solution (pH 3.0), and leaving the mixture to stand for 72 hours at 30 °C. Thereafter, the mixture was neutralized with a four-fold dilution with 1 M Tris-hydrochloric acid buffer solution (pH 7.0) and the antibody binding activity of the Fc-binding protein was measured using the ELISA method described in section (4) of Example 2.
(4) The residual activity was calculated by dividing the antibody binding activity when acid treatment was performed by the antibody binding activity when the acid treatment was not performed, and acid stability was evaluated.

The results are illustrated in Table 7. Since FcγRIIa-m11-Del1 and FcγRIIa-m11-Del5 had a higher residual activity than FcγRIIa-m11 72 hours after treatment, it was confirmed that the acid stability of these proteins had been improved compared to FcγRIIa-m11.

### [Table 7]

**Table 7**

| EXAMPLE | NAME | SEQ ID NO | RESIDUAL ACTIVITY [%] |
|---|---|---|---|
| EXAMPLE 7 (a) | FcγRIIa-m11-Del1 | 35 | 89.8 |
| EXAMPLE 7 (b) | FcγRIIa-m11-Del2 | 37 | 81.3 |
| EXAMPLE 7 (c) | FcγRIIa-m11-Del3 | 39 | 72.4 |
| EXAMPLE 7 (d) | FcγRIIa-m11-Del4 | 41 | 76.7 |
| EXAMPLE 7 (c) | FcγRIIa-m11-Del5 | 43 | 86. 5 |
| EXAMPLE 7 (f) | FcγRIIa-m11-Del6 | 45 | 25.4 |
| EXAMPLE 7(g) | FcγRIIa-m11-Del7 | 47 | 33.3 |
| EXAMPLE 7(h) | FcγRIIa-m11-Del8 | 49 | - |
| EXAMPLE 7(i) | FcγRIIa-m11-Del9 | 51 | - |
| EXAMPLE 7(j) | FcγRIIa-m11-Del10 | 53 | - |
| EXAMPLE 6 | FcγRIIa-m11 | 25 | 83.6 |

## Claims

1. An FcyRIIa protein comprising at least amino acid residues from glutamine at position 29 to glutamine at position 201 of the amino acid sequence set forth in SEQ ID NO: 2, provided the FcyRIIa protein has a substitution of leucine to serine at position 190 of SEQ ID NO: 2, and amino acid substitutions at the 6 positions indicated below:
a substitution of isoleucine to valine at position 68 of SEQ ID NO: 2,
a substitution of histidine to glutamine at position 80 of SEQ ID NO: 2,
a substitution of serine to threonine at position 84 of SEQ ID NO: 2,
a substitution of asparagine to threonine at position 90 of SEQ ID NO: 2,
a substitution of asparagine to serine at position 91 of SEQ ID NO: 2,
a substitution of histidine to arginine at position 125 of SEQ ID NO: 2.

2. The FcyRIIa protein according to claim 1 selected from (iv), (vii) or (x) below:
(iv) an FcyRIIa protein comprising at least amino acid residues from glutamine at position 29 to glutamine at position 201 of an amino acid sequence set forth in any of SEQ ID NOs: 5, 13, 17, 19, 23, 25;
(vii) an FcyRIIa protein comprising at least amino acid residues from glutamine at position 29 to glutamine at position 200 of the amino acid sequence set forth in SEQ ID NO: 35;
(x) an FcyRIIa protein comprising at least amino acid residues from glutamine at position 29 to glutamine at position 196 of the amino acid sequence set forth in SEQ ID NO: 43.

3. A polynucleotide encoding the FcyRIIa protein according to any one of claims 1 or 2.

4. An expression vector comprising the polynucleotide according to claim 3.

5. A transformant comprising the recombinant vector according to claim 4 and capable of producing an FcyRIIa protein.

6. The transformant according to claim 5, wherein the host is *Escherichia coli.*

7. A method for producing an FcyRIIa protein, comprising the steps of: producing an FcyRIIa protein by culturing the transformant according to claim 5 or 6; and harvesting the FcyRIIa protein from the obtained culture product.

8. An antibody adsorbent comprising immobilized FcγRIIa protein according to any one of claims 1 or 2 on an insoluble carrier.

9. An antibody separation method comprising the steps of: adding a solution containing an antibody to a column filled with the adsorbent according to claim 8 and adsorbing the antibody to the adsorbent; and eluting the antibody adsorbed to the adsorbent using an eluent.

## Patentansprüche

1. FcyRIIa-Protein, aufweisend mindestens Aminosäurereste von Glutamin an Position 29 bis Glutamin an Position 201 der Aminosäuresequenz wie in SEQ ID NO: 2 dargelegt, vorausgesetzt, dass das FcyRIIa-Protein eine Substitution von Leucin zu Serin an Position 190 von SEQ ID NO: 2 und Aminosäuresubstitutionen an den nachstehend angegebenen 6 Positionen hat:
eine Substitution von Isoleucin zu Valin an Position 68 von SEQ ID NO: 2,
eine Substitution von Histidin zu Glutamin an Position 80 von SEQ ID NO: 2,
eine Substitution von Serin zu Threonin an Position 84 von SEQ ID NO: 2,
eine Substitution von Asparagin zu Threonin an Position 90 von SEQ ID NO: 2,
eine Substitution von Asparagin zu Serin an Position 91 von SEQ ID NO: 2,
eine Substitution von Histidin zu Arginin an Position 125 von SEQ ID NO: 2.

2. FcyRIIa-Protein nach Anspruch 1, ausgewählt aus den nachstehenden (iv), (vii) oder (x):
(iv) einem FcyRIIa-Protein, aufweisend mindestens Aminosäurereste von Glutamin an Position 29 bis Glutamin an Position 201 einer Aminosäuresequenz wie in SEQ NO ID: 5, 13, 17, 19, 23, 25 dargelegt;
(vii) einem FcyRIIa-Protein, aufweisend mindestens Aminosäurereste von Glutamin an Position 29 bis Glutamin an Position 200 der Aminosäuresequenz wie in SEQ ID NO: 35 dargelegt;
(x) einem FcyRIIa-Protein, aufweisend mindestens Aminosäurereste von Glutamin an Position 29 bis Glutamin an Position 196 der Aminosäuresequenz wie in SEQ ID NO: 43 dargelegt.

3. Polynukleotid, welches das FcyRIIa-Protein nach einem der Ansprüche 1 oder 2 kodiert.

4. Expressionsvektor, der das Polynukleotid nach Anspruch 3 aufweist.

5. Transformant, der den rekombinanten Vektor nach Anspruch 4 aufweist und in der Lage ist, ein FcyRIIa-Protein herzustellen.

6. Transformant nach Anspruch 5, wobei der Wirt *Escherichia coli* ist.

7. Verfahren zum Herstellen eines FcγRIIa-Proteins, aufweisend die folgenden Schritte: Herstellen eines FcγRIIa-Proteins durch Kultivieren des Transformanten nach Anspruch 5 oder 6; und Ernten des FcγRIIa-Proteins aus dem gewonnenen Kulturprodukt.

8. Antikörper-Adsorptionsmittel, aufweisend ein immobilisiertes FcγRIIa-Protein nach einem der Ansprüche 1 oder 2 auf einem unlöslichen Träger.

9. Verfahren zur Abtrennung eines Antikörpers, aufweisend die folgenden Schritte: Zugeben einer einen Antikörper enthaltenden Lösung zu einer Säule, die mit dem Adsorptionsmittel nach Anspruch 8 gefüllt ist, und Adsorbieren des Antikörpers an das Adsorptionsmittel und Eluieren des an das Adsorptionsmittel adsorbierten Antikörpers unter Verwendung eines Eluenten.

## Revendications

1. Protéine FcγRIIa comprenant au moins les résidus d'acides aminés de la glutamine en position 29 à la glutamine en position 201 de la séquence d'acides aminés présentée dans SEQ ID NO : 2, à condition que la protéine FcγRlla ait une substitution de la leucine à la sérine en position 190 de SEQ ID NO : 2, et des substitutions d'acides aminés aux 6 positions indiquées ci-dessous :
une substitution de l'isoleucine à la valine en position 68 de SEQ ID NO : 2,
une substitution de l'histidine à la glutamine à la position 80 de SEQ ID NO : 2,
une substitution de la sérine à la thréonine à la position 84 de SEQ ID NO : 2,
une substitution de l'asparagine à la thréonine à la position 90 de SEQ ID NO : 2,
une substitution de l'asparagine à la sérine à la position 91 de SEQ ID NO : 2,
une substitution de l'histidine à l'arginine à la position 125 de SEQ ID NO : 2.

2. La protéine FcγRIIa selon la revendication 1 choisie parmi (iv), (vii) ou (x) ci-dessous :
(iv) une protéine FcγRlla comprenant au moins les résidus d'acides aminés de la glutamine en position 29 à la glutamine en position 201 d'une séquence d'acides aminés présentée dans l'un quelconque des SEQ ID NO : 5, 13, 17, 19, 23, 25 ;
(vii) une protéine FcγRlla comprenant au moins les résidus d'acides aminés de la glutamine en position 29 à la glutamine en position 200 de la séquence d'acides aminés présentée dans SEQ ID NO : 35 ;
(x) une protéine FcγRlla comprenant au moins les résidus d'acides aminés de la glutamine en position 29 à la glutamine en position 196 de la séquence d'acides aminés présentée dans SEQ ID NO : 43.

3. Polynucléotide encodant la protéine FcγRIIa selon l'une quelconque des revendications 1 ou 2.

4. Vecteur d'expression comprenant le polynucléotide selon la revendication 3.

5. Transformant comprenant le vecteur recombinant selon la revendication 4 et capable de produire une protéine FcγRIIa.

6. Transformant selon la revendication 5, dans lequel l'hôte est *Escherichia coli.*

7. Méthode de production d'une protéine FcγRIIa, comprenant les étapes suivantes : production d'une protéine FcγRIIa par culture du transformant selon la revendication 5 ou 6 ; et récolte de la protéine FcγRIIa à partir du produit de culture obtenu.

8. Adsorbant d'anticorps comprenant la protéine FcγRIIa immobilisée selon l'une quelconque des revendications 1 ou 2 sur un support insoluble.

9. Méthode de séparation d'anticorps comprenant les étapes d' : ajout d'une solution contenant un anticorps dans une colonne remplie de l'adsorbant selon la revendication 8 et adsorption de l'anticorps par l'adsorbant ; et d'élution de l'anticorps adsorbé par l'adsorbant à l'aide d'un éluant.
